(19) Europäisches Patentamt
European Patent Office
Office européen des brevets

(11) Publication number: **0 059 989**
**A1**

(12) # EUROPEAN PATENT APPLICATION

(21) Application number: 82102714.1

(22) Date of filing: 11.03.80

(51) Int. Cl.³: C 07 D 221/22
//C07D211/70, A61K31/435

(30) Priority: 12.03.79 US 19527

(43) Date of publication of application:
15.09.82 Bulletin 82/37

(84) Designated Contracting States:
DE GB NL SE

(60) Publication number of the earlier application in accordance with Art. 76 EPC: 0 018 077

(71) Applicant: ELI LILLY AND COMPANY
307, East McCarty Street
Indianapolis Indiana 46285(US)

(72) Inventor: Zimmerman, Dennis Michael
131, Thorncrest Drive
Mooresville Indiana 46158(US)

(74) Representative: Crowther, Terence Roger et al,
Erl Wood Manor
Windlesham Surrey GU20 6PH(GB)

(54) **2,3- and 3,4-dehydro-5-phenylmorphans and their preparation.**

(57) Compounds of formulae

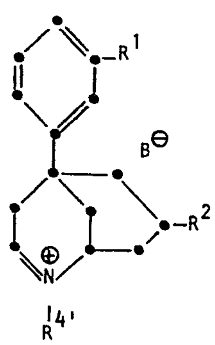

III and

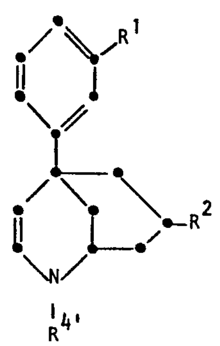

IV

having use as intermediates in the preparation of analgesic compounds are prepared by reacting a 1, 2, 5, 6-tetrahydro-4-phenylpyridine compound of the formula

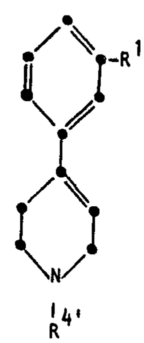

I

with a propenyl alkylating agent of the formula

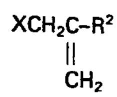

wherein:

$R^1$ is hydrogen, hydroxy or $C_1$-$C_3$ alkoxy;

$R^2$ is hydrogen, $C_1$-$C_5$ alkyl or $CH_2C_2$-$C_4$ alkenyl;

$R^{4'}$ is hydrogen, $C_1$-$C_{10}$ alkyl, phenyl $C_1$-$C_3$ alkyl, or $CH_2C_3$-$C_6$ cycloalkyl; and X is a good leaving group in the presence of a strong base to provide a 1,4,5,6-tetrahydro-4-

./...

EP 0 059 989 A1

phenyl-4-(2-propenyl)-pyridine compound of the formula

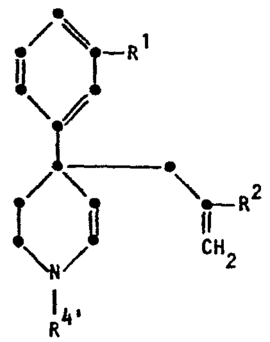

II

wherein:

$R^1$, $R^2$ and $R^{4'}$ are the same as above and reacting said compound with a protonic acid of the formula HB′, in which B′ is an anion B or B″ wherein the product is recovered as the salt in which B′ is the anion B or as the free base of a salt in which B is exchanged for the anion B″.

## 2,3- AND 3,4-DEHYDRO-5-PHENYLMORPHANS AND THEIR PREPARATION.

This invention relates to intermediates useful in the preparation of 5-phenylmorphans, which have analgesic activity. A process for the preparation of 5-phenylmorphans from 1,2,5,6-tetrahydro-4-phenyl-pyridines is disclosed in our co-pending application No. 80300746.7.

The invention described therein provides a 5-phenylmorphan compound having the formula

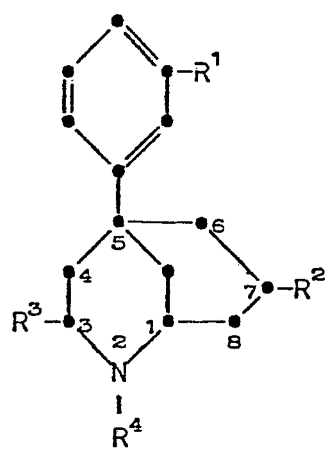

VI

wherein:

$R^1$ is hydrogen, hydroxy or $C_1$-$C_3$ alkoxy

$R^2$ abd $R^3$ independently are hydrogen, $C_1$-$C_5$ alkyl or $CH_2C_2$-$C_4$ alkenyl; except that $R^2$ and $R^3$ both are not hydrogen;

$R^4$ is hydrogen, $C_1$-$C_{10}$ alkyl, phenyl $C_1$-$C_3$ alkyl, $CH_2C_2$-$C_9$ alkenyl, or $CH_2C_3$-$C_6$ cycloalkyl; and

the pharmaceutically acceptable acid addition salts thereof.

Preferred compounds are those wherein $R^1$ is hydrogen, hydroxy or methoxy; $R^2$ and $R^3$ independently are hydrogen or n-$C_1$-$C_5$ alkyl; and $R^4$ is n-$C_1$-$C_{10}$ alkyl, benzyl, 2-phenethyl, $CH_2C_2$-$C_3$ alkenyl and $CH_2C_3$-$C_4$ cycloalkyl.

That invention also provides pharmacuetical formulations useful in the treatment of pain comprising an analgesically effective amount of a 5-phenylmorphan of the above formula VI in combination with a suitable pharmaceutical carrier.

This invention provides a 2,3-dehydro-5-phenylmorphan compound of the formula

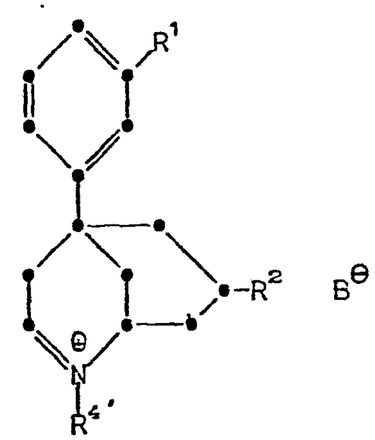

III

wherein:

$R^1$ is hydrogen, hydroxy or $C_1$-$C_3$ alkoxy;

$R^2$ is hydrogen, $C_1$-$C_5$ alkyl or $CH_2C_2$-$C_4$ alkenyl;

$R^{4'}$ is hydrogen, $C_1$-$C_{10}$ alkyl, phenyl $C_1$-$C_3$ alkyl, or $CH_2C_3$-$C_6$ cycloalkyl; and

$B^{\ominus}$ is an anion.

The invention also provides a 3,4-dehydro-5-phenylmorphan compound of the formula

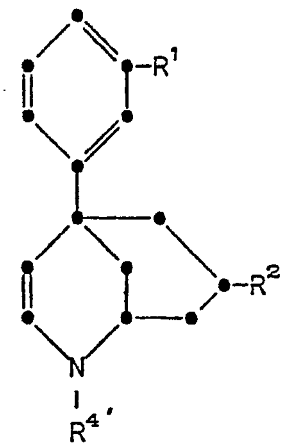

IV

wherein:

$R^1$ is hydrogen, hydroxy or $C_1$-$C_3$ alkoxy;

$R^2$ is hydrogen, $C_1$-$C_5$ alkyl or $CH_2C_2$-$C_4$ alkenyl; and

$R^{4'}$ is hydrogen, $C_1$-$C_{10}$ alkyl, phenyl $C_1$-$C_3$ alkyl or $CH_2C_3$-$C_6$ cycloalkyl.

This invention also provides a process for preparing a 2,3- or 3,4-dehydro-5-phenylmorphan compound of the formulae

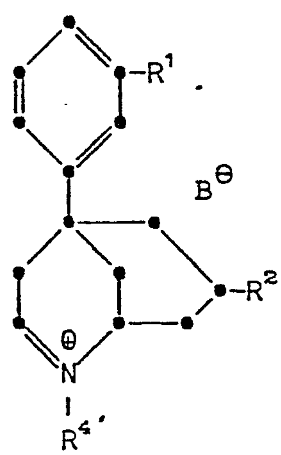

III and

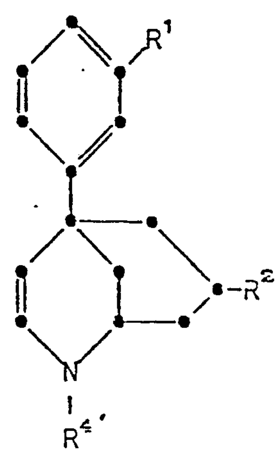

IV

wherein:

$R^1$ is hydrogen, hydroxy or $C_1$-$C_3$ alkoxy;

$R^2$ is hydrogen, $C_1$-$C_5$ alkyl or $CH_2C_2$-$C_4$ alkenyl;

$R^{4'}$ is hydrogen or $C_1$-$C_{10}$ alkyl, phenyl $C_1$-$C_3$ alkyl or $CH_2C_3$-$C_6$ cycloalkyl; and

B is an anion characterized by reacting a 1,2,5,6-tetrahydro-4-phenylpyridine compound of the formula

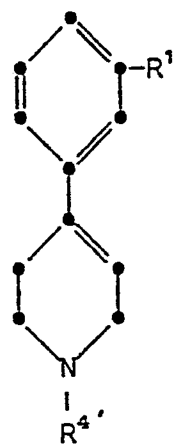

I

with a propenyl alkylating agent of the formula

$$\begin{array}{l} XCH_2C-R^2 \\ \qquad\;\| \\ \qquad CH_2 \end{array}$$

wherein:

$R^1$ is hydrogen, hydroxy or $C_1$-$C_3$ alkoxy;

$R^2$ is hydrogen, $C_1$-$C_5$ alkyl or $CH_2C_2$-$C_4$ alkenyl;

$R^{4'}$ is hydrogen, $C_1$-$C_{10}$ alkyl, phenyl $C_1$-$C_3$ alkyl, or $CH_2C_3$-$C_6$ cycloalkyl; and X is a good leaving group;

in the presence of a strong base to provide a 1,4,5,6-tetrahydro-4-phenyl-4-(2-propenyl)-pyridine compound of the formula

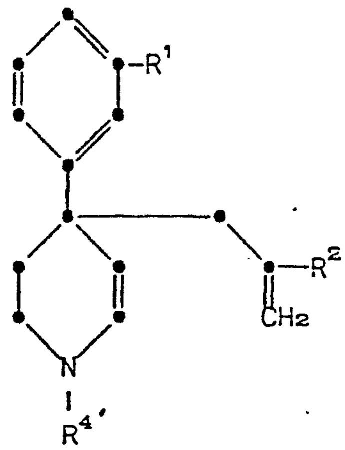

II

wherein:

$R^1$, $R^2$ and $R^{4'}$ are the same as above and reacting said compound with a protonic acid of the formula HB', in which B' is an anion B or B" wherein the product is recovered as the salt in which B' is the anion B or as the free base of a salt in which B is exchanged for the anion B".

As used throughout this specification and in the appended claims, $R^1$ includes "$C_1$-$C_3$ alkoxy" such as methoxy, ethoxy and n-propoxy. A preferred alkoxy group is methoxy.

$R^2$ and $R^3$ are defined to include "$C_1$-$C_5$ alkyl" groups such as methyl, ethyl, n-propyl, isopropyl, n-butyl, n-pentyl and isopentyl, as well as "$CH_2C_2$-$C_4$ alkenyl" groups such as allyl, 3-butenyl, 2-pentenyl, 2-methyl-2-butenyl and the like.

$R^4$ as defined herein includes "$C_1$-$C_{10}$ alkyl" groups such as methyl, ethyl, n-pentyl, isohexyl, 2-methylheptyl, 1,1-dimethylheptyl, 2-ethyloctyl, n-nonyl, n-decyl, and related alkyl groups. $R^4$ also includes

"$C_1$-$C_3$ phenylalkyl groups such as benzyl, 2-phenethyl and 3-phenylpropyl. The term "$CH_2C_2$-$C_9$ alkenyl" refers to groups such as allyl, 3-butenyl, 3-pentenyl, 4-hexenyl, 2,3-dimethyl-2-pentenyl, 3-octenyl, 5-decenyl and the like. $R^4$ additionally includes "$CH_2C_3$-$C_6$ cycloalkyl" groups such as cyclopropylmethyl, cyclobutylmethyl, cyclopentylmethyl and cyclohexymethyl. Cyclopropylmethyl is a preferred cycloalkyl substituent.

The phenylmorphan compounds are prepared by reacting a 1-alkyl-4-phenyl-1,2,5,6-tetrahydropyridine of Formula I with a strong base such as butyl lithium or phenyl lithium and a propenyl alkylating agent such as allyl bromide to provide a 1-alkyl-4-phenyl-4-(2-propenyl)-1,4,5,6-tetrahydropyridine of Formula II. The latter compound is reacted with a protonic acid to effect ring closure with concomitant double bond migration to give a 2-alkyl-2,3-dehydro-5-phenylmorphanium salt of Formula III of the invention. Reduction of the double bond of such salt affords the corresponding 3-unsubstituted phenylmorphan of Formula V, whereas alkylation of the 2,3-dehydro-5-phenylmorphanium salt provides the corresponding 3-substituted-phenylmorphan of Formula VI. The overall reaction is depicted in the following general scheme:

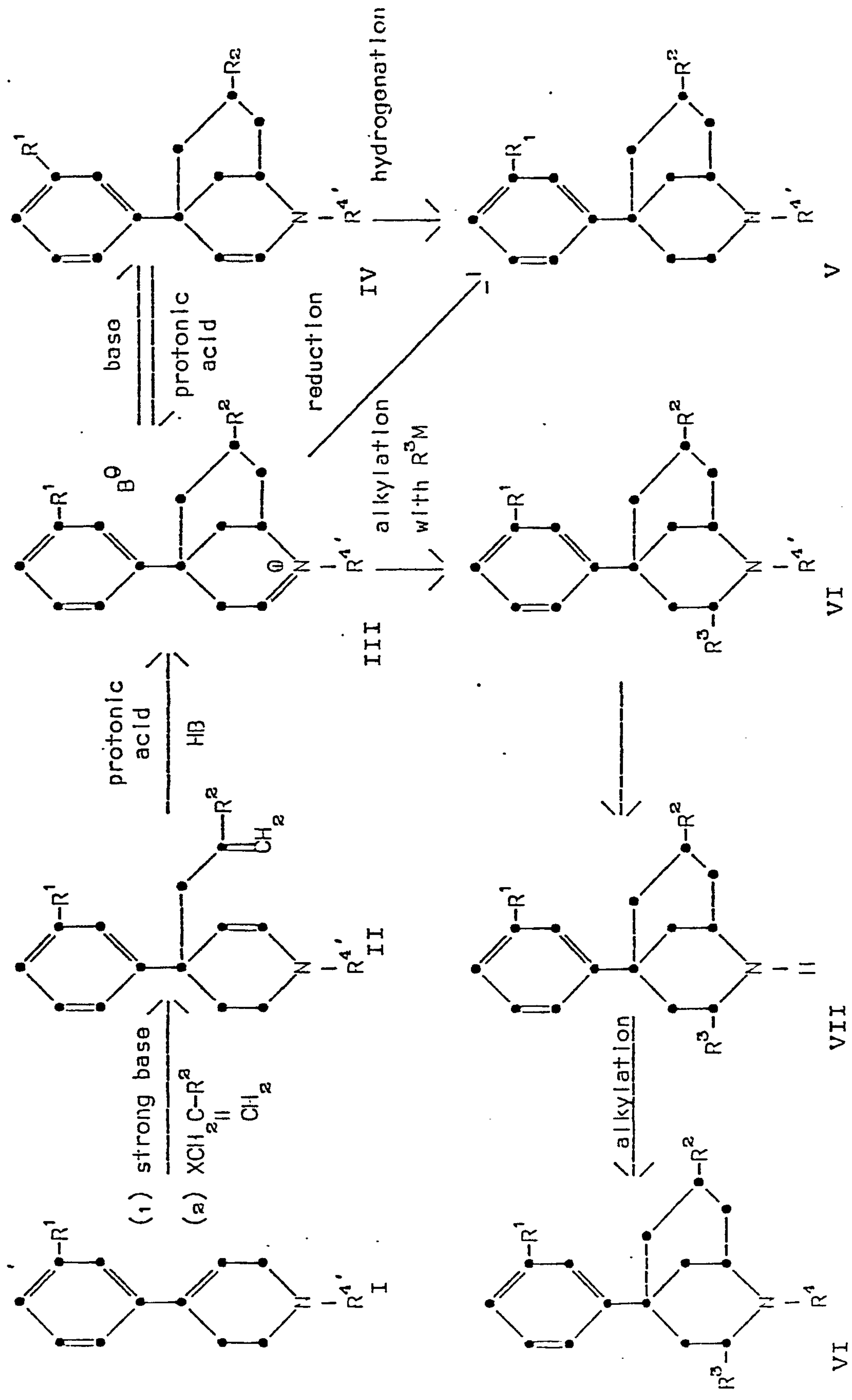
base
protonic acid
reduction
hydrogenation
alkylation with $R^3M$
protonic acid
HB
(1) strong base
(2) $XCH_2C(=CH_2)-R^2$
alkylation
I
II
III
IV
V
VI
VII
VI

In the above scheme, $R^1$, $R^2$, $R^3$ and $R^{4'}$ are as defined hereinbefore, X is a leaving group and B and M are ionic radicals. It should be noted that when the above reactions are carried out wherein $R^1$ is methoxy, $R^2$ and $R^3$ both are hydrogen and $R^{4'}$ is methyl, the compound prepared is one disclosed by Rogers et al. in J. Med. Chem. 17, 1328 (1974), and is a precursor to the potent analgesic 2-methyl-5-(3-hydroxyphenyl)morphan. The process provided herein is an improvement over the prior art processes for preparing phenylmorphans since the product is produced in over fifty percent yield whereas the prior art processes produce the product in less than ten percent overall yield.

The first step in the process of this invention is the reaction of a 4-phenyl-1,2,5,6-tetrahydropyridine of Formula I with a strong base and a propenyl alkylating agent. Strong bases commonly utilized in the reaction include lower alkyl metalides such as methyl lithium, methyl sodium, *n*-propyl potassium, *n*-butyl lithium, as well as amides such as lithium diisopropylamide, sodium amide, lithium diethylamide, and hydrides such as sodium hydride. Typical propenyl alkylating agents include allyl bromide, allyl iodide, 2-methylallyl bromide, 2-ethylallyl *p*-toluenesulfonate, 2-*n*-propylallyl azide, 2-ethenylallyl iodide, 2-(2-propenyl)allyl azide, 2-(*n*-pentyl)allyl bromide and the like. The alkylation of the 4-phenyl-1,2,5,6-tetrahydropyridine of Formula I is carried out by first reacting the pyridine derivative with about a 1 to 20 molar excess of a strong base in an unreactive organic

solvent such as diethyl ether, tetrahydrofuran, dicxane, dichloromethane, benzene or the like. The reaction commonly is carried out at a reduced temperature of from 10 to -60°C. The tetrahydropyridine and the strong base are simply mixed together in a suitable solvent and stirred for 10 to 20 minutes, and then the reaction mixture is added to a solution containing about an equimolar amount or an excess of the appropriate propenyl alkylating agent in a suitable unreactive solvent such as diethyl ether, dicxane, or the like. The alkylation reaction typically is complete within 10 minutes to 2 hours when carried out at a temperature of from 25 to -60°C. The product is readily isolated by simply adding water or brine to the reaction mixture, separating the organic layer and then removing the organic solvent, for instance by evaporation under reduced pressure. The product, a 1-alkyl-4-allyl (or 2-alkylallyl or 2-alkenylallyl)-4-phenyl-1,4,5,6-tetrahydropyridine of Formula II, can be further purified if desired by conventional methods such as distillation, chromatography and the like.

The next step in the process comprises reacting the 1-alkyl-4-allyl (or 2-alkylallyl or 2-alkenylallyl)-4-phenyl-1,4,5,6-tetrahydropyridine of Formula II with a protonic acid to effect cyclization and concomitant double bond migration to provide a 2-alkyl-2,3-dehydro-5-phenylmorphanium salt of Formula III. Any of a number of protonic acids can be utilized to effect the cyclization and double bond migration. Commonly used acids include phosphoric acid, tetrafluoroboric acid, hydrochloric acid, sulfuric acid, nitric acid, *para*-toluenesulfonic acid, and related protonic acids. Phosphoric acid is a preferred protonic acid. The cyclization reaction generally is carried out in a solvent, typically an acidic solvent such as formic acid, acetic acid, sulfuric acid, hydrochloric acid or the like. Non-acidic solvents which can be used include dioxane, tetrahydrofuran and N,N-dimethylformamide. A preferred solvent is formic acid.

The allyl substituted tetrahydro pyridine typically is dissolved in an excess of protonic acid such as phosphoric acid in a suitable solvent such as formic acid. The reaction can be carried out at a temperature from 0°C. to 50°C., and routinely is carried out at 20°C. to 30°C. The cyclization routinely is complete within about 24 to about 72 hours. As noted in the above mechanistic scheme, the product from such protonic acid cyclization reaction is a salt, namely a 2-alkyl-2,3-dehydro-5-phenyl-7-(unsubstituted or substituted) morphanium salt of Formula III. Such salt can readily be isolated by simply removing the reaction solvent and recrystallizing the salt from common solvents such as ethyl acetate, ethanol, and the like. An alternative method for obtaining the salt of the invention in a purified form comprises first making the acidic reaction mixture basic, for instance by adding a base such as sodium hydroxide, potassium hydroxide, sodium ethoxide or butyl lithium, thereby converting the 2,3-dehydrophenylmorphanium salt of Formula III to a free base of Formula IV according to the following scheme:

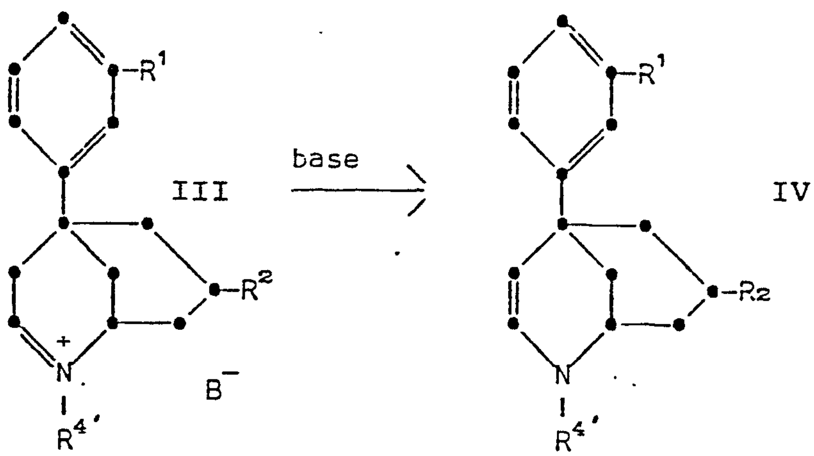


wherein $R^1$, $R^2$ and $R^{4'}$ are as defined above. The 3,4-dehydrophenylmorphan free base of the invention thus formed is readily isolated by simply extracting the alkaline reaction mixture with a water immiscible solvent such as diethyl ether or chloroform, and then removing the organic solvent by evaporation. Reaction of such free base with a protonic acid converts it back again to the corresponding 2,3-dehydrophenylmorphanium salt of Formula III. Examples of typical 2-alkyl-2,3-dehydro-5-phenylmorphanium salts and 2-alkyl-3,4-dehydro-5-phenylmorphans thus prepared include the following:

2-methyl-2,3-dehydro-5-phenylmorphanium tetrafluoroborate;

2-methyl-2,3-dehydro-5-(3-methoxyphenyl)-7-methylmorphanium bromide;

2-*isopropyl*-2,3-dehydro-5-(3-ethoxyphenyl)-7-ethylmorphanium perchlorate;

2-cyclopropylmethyl-2,3-dehydro-5-phenyl-7-*n*-pentylmorphanium acetate;

2-benzyl-2,3-dehydro-5-(3-methoxyphenyl)-7-*n*-butylmorphanium sulfate;

2-methyl-3,4-dehydro-5-phenylmorphan;

2-ethyl-3,4-dehydro-5-(3-methoxyphenyl)-morphan;

2-*iso*propyl-3,4-dehydro-5-(3-ethoxyphenyl)-morphan;

2-cyclopropylmethyl-3,4-dehydro-5-(3-hydroxyphenyl)morphan;

2-benzyl-3,4-dehydro-5-phenyl-7-methyl-morphan;

2-methyl-3,4-dehydro-5-(3-methoxyphenyl)-7-ethylmorphan;

2-*n*-heptyl-3,4-dehydro-5-phenyl-7-*n*-pentylmorphan; and

2-(2-phenylethyl)-3,4-dehydro-5-(3-methoxyphenyl)-7-(3-butenyl)morphan.

The phenylmorphans which are substituted at the 3-position with an alkyl or alkenyl group are prepared by alkylation of a 2-substituted-2,3-dehydro-5-phenylmorphanium salt of the invention. Alkylating agents utilized in the reaction are defined by the formula $R^3M$ wherein $R^3$ is $C_1$-$C_5$ alkyl or $CH_2C_2$-$C_4$ alkenyl and M is a cationic radical. Commonly utilized alkylating agents include alkali metal alkyl or alkenyl metalides such as methyl lithium, ethyl sodium, *n*-butyl lithium, *iso*pentyl potassium, 2-propenyl lithium, 3-butenyl sodium and related alkyl or alkenyl metalides. Additional alkylating agents which can be used are Grignard reagents of the formula

$R^3$ Mg halide, such as methyl magnesium bromide and n-propyl magnesium iodide, as well as dialkyl cuprates such as diethyl cuprate and diallyl cuprate.

The alkylation reaction preferably is carried out by combining the appropriate 2-substituted-2,3-dehydro-5-phenyl-(7-substituted or unsubstituted)-morphanium salt of Formula III with an equimolar amount or excess of alkylating or alkenylating agent in an unreactive organic solvent such as diethyl ether, diisopropyl ether, dioxane, tetrahydrofuran or the like. The reaction generally is complete within two to ten hours when carried out at 20 to 40°C. The product, a 2,3-disubstituted-5-phenyl-(7-substituted or unsubstituted)morphan of Formula VI, is isolated by diluting the reaction mixture with aqueous ammonium chloride and then washing the organic layer several times with water. Separation of the organic layer and evaporation of the solvent therefrom then provides the product as a solid or an oil. Further purification can be accomplished if desired by routine methods such as column chromatography, crystallization, salt formation and the like.

The phenylmorphans which are unsubstituted at the 3-position can be prepared by reduction of a 2,3-dehydro-5-phenylmorphanium salt of Formula III. For example, a salt such as 2-benzyl-2,3-dehydro-5-(3-ethoxyphenyl)-7-ethylmorphanium tetrafluoroborate can be reacted with about an equimolar quantity or excess of a reducing agent such as sodium borohydride or lithium aluminum hydride to provide the corresponding saturated phenylmorphan, i.e.

2-benzyl-5-(3-ethoxyphenyl)-7-ethylmorphan.

Such 3-unsubstituted phenylmorphans can alternatively be prepared by catalytic hydrogenation of the aforementioned 3,4-dehydro-5-phenylmorphans. For example, a compound such as 2-cyclopropylmethyl-3,4-dehydro-5-(3-hydroxyphenyl)-7-isobutylmorphan can be hydrogenated in the presence of a suitable catalyst to provide 2-cyclopropylmethyl-5-(3-hydroxyphenyl)-7-isobutylmorphan. The catalytic hydrogenation reactions typically are carried out in organic solvents such as methanol or ethanol, and with common catalysts such as palladium on carbon, platinum, Raney nickel and the like. When the reaction is carried out at 20 to 50°C. with a hydrogen pressure of 30 to 80 psi, the reduction is substantially complete after one-half to twenty-four hours. The reduced product is readily isolated by simply filtering the reaction mixture and then removing the reaction solvent. The phenylmorphan thus formed can be further purified if desired by routine methods such as crystallization, chromatography and salt formation.

The following detailed examples are provided to illustrate various specific aspects of the invention.

Preparation 1

1-Methyl-4-allyl-4-(3-methoxyphenyl)-1,4,5,6-tetrahydropyridine

A solution of 74 ml. of 1.6 M n-butyl lithium was added dropwise to a cold (0°C.) stirred solution of 24.36 g. of 1-methyl-4-(3-methoxyphenyl)-1,2,5,6-tetrahydropyridine in 300 ml. of tetrahydrofuran. The reaction mixture was stirred for 10 minutes at 0°C. and then added dropwise over thirty minutes to a stirred cold

(-50°C.) solution of allyl bromide in 250 ml. of diethyl ether. The reaction mixture was warmed to 0°C. and then diluted with 500 ml. of aqueous sodium chloride solution. The organic layer was separated, diluted with an additional 2 liters of diethyl ether, washed with water and dried. Removal of the solvent by evaporation under reduced pressure provided 33 g. of the crude product as an oil. The oil was distilled twice to provide 17.91 g. of 1-methyl-4-allyl-4-(3-methoxyphenyl)-1,4,5,6-tetrahydropyridine. B.P. 120-123°C. at 0.1 torr.

Analysis calc. for $C_{16}H_{21}NO$

Theory: C, 78.97; H, 8.70; N, 5.76.

Found: C, 78.72; H, 8.55; N, 5.48.

Example 1

2-Methyl-3,4-dehydro-5-(3-methoxyphenyl)morphan

A solution of 1.0 g. of 1-methyl-4-allyl-4-(3-methoxyphenyl)-1,4,5,6-tetrahydropyridine dissolved in 2.5 ml. of 85.8 percent aqueous phosphoric acid and 2.5 ml. of formic acid was stirred for sixty-six hours at 24°C. under a nitrogen atmosphere. The reaction mixture then was diluted with 150 ml. of ice water and made alkaline by the addition of 50 percent aqueous sodium hydroxide. The product was extracted from the alkaline solution into diethyl ether. The ethereal extracts were combined, washed with water and dried. Removal of the solvent by evaporation under reduced pressure afforded 950 mg. of 2-methyl-3,4-dehydro-5-(3-methoxyphenyl)morphan.

Mass spec. Theory 243, Found 243.

## Example 2

2-Methyl-5-(3-methoxyphenyl)morphan

A solution of 4.0 g. of 2-methyl-3,4-dehydro-5-(3-methoxyphenyl)morphan in 150 ml. of ethanol containing 1.5 g. of five percent palladium on carbon was stirred for eighteen hours at 24°C. under a hydrogen pressure of 60 psi. The reaction mixture then was filtered and the solvent was evaporated from the filtrate to provide 3.72 g. of 2-methyl-5-(3-methoxyphenyl)morphan.

The product thus obtained was dissolved in 60 ml. of isopropanol and the solution was saturated with hydrogen bromide to form 3.22 g. of a white precipitate. The precipitated salt was recrystallized from 20 ml. of diisopropyl ether and 40 ml. of isopropanol to provide 1.0 g. of 2-methyl-5-(3-methoxyphenyl)-morphanium bromide. M.P. 152.5-154°C.

Analysis calc. for $C_{16}H_{24}BrNO$

Theory: C, 58.90; H, 7.41; N, 4.29.

Found: C, 59.19; H, 7.13; N, 4.04.

Preparation 2

1-Methyl-4-(3-methoxyphenyl)-4-(2-methylallyl)-1,4,5,6-tetrahydropyridine

A solution of 74 ml. of 1.6 molar n-butyl lithium in tetrahydrofuran was added dropwise to a cold stirred solution of 24.36 g. of 1-methyl-4-(3-methoxyphenyl)-1,2,5,6-tetrahydropyridine in 300 ml. of tetrahydrofuran. After the addition was complete and the reaction mixture had been stirred at 0°C. for ten minutes, the mixture was added dropwise over thirty minutes to a stirred cold (-50°C.) solution of 10.86 g. of 3-chloro-2-methylpropene (methallyl chloride) in 250 ml. of diethyl ether. The reaction mixture was stirred and allowed to warm slowly to 0°C., at which time the reaction mixture was diluted by the dropwise addition of 500 ml. of saturated aqueous sodium chloride solution. The organic layer next was separated, diluted with 2 liters of fresh diethyl ether, washed with fresh water and dried. Removal of the solvent by evaporation under reduced pressure then provided 33.43 g. of an oil, which after distillation gave 20.97 g. of 1-methyl-4-(3-methoxyphenyl)-4-(2-methylallyl)-1,4,5,6-tetrahydropyridine. B.P. 138-141°C. at 0.1 torr. Mass spec. Theory: 257; Found $M^+$ 257.

Example 3

2,7-Dimethyl-2,3-dehydro-5-(3-methoxyphenyl)morphanium perchlorate

Twenty grams of 1-methyl-4-(3-methoxyphenyl)-4-(2-methylallyl)-1,4,5,6,-tetrahydropyridine were dissolved in 50 ml. of 85.8% aqueous phosphoric acid containing 50 ml. of formic acid. The reaction mixture was stirred for forty-eight hours at ambient temperature, and then diluted by the addition of 300 ml. of ice water. Fifty percent aqueous sodium hydroxide was added to the aqueous reaction mixture to pH 11, and then the product was extracted therefrom into diethyl ether. The ethereal extracts were combined, washed with water and dried. Removal of the solvent by evaporation under reduced pressure afforded 21 g. of crude oil, which after distillation gave 17.68 g. of 2,7-dimethyl-3,4-dehydro-5-(3-methoxyphenyl)morphan. B.P. 130-138°C. at 0.1 torr.

Excess perchloric acid was added to a solution of 12.68 g. of the compound thus prepared dissolved in diethyl ether. The precipitated salt which formed was collected and recrystallized from 200 ml. of ethanol to afford 12.82 g. of 2,7-dimethyl-2,3-dehydro-5-(3-methoxyphenyl)morphanium perchlorate. M.P. 131-133°C.

Example 4

2,7-Dimethyl-5-(3-methoxyphenyl)morphan

A solution containing 5 g. of 2,7-dimethyl-3,4-dehydro-5-(3-methoxyphenyl)morphan dissolved in 200 ml. of ethanol containing 5 g. of five percent palladium on carbon was stirred for twenty-four hours at about 24°C. under hydrogen at 60 psi. The reaction mixture then was filtered and the solvent was removed from the filtrate by evaporation to give 4.8 g. of an oil. The oil was then dissolved in 100 ml. of diethyl ether and the solution was saturated with hydrogen bromide to effect salt formation. The salt precipitated out of solution and was collected and recrystallized from 30 ml. of diisopropyl ether and 25 ml. of isopropanol to provide 1.78 g. of 2,7-dimethyl-5-(3-methoxyphenyl)morphanium bromide. M.P. 178-181°C.

Analysis calc. for $C_{17}H_{26}BrNO$

Theory: C, 60.00; H, 7.70; N, 4.12.

Found: C, 59.74; H, 7.47; N, 4.38.

Example 5

2,3,7-Trimethyl-5-(3-methoxyphenyl)morphan

To a stirred solution of 60 ml. of 1.6 molar methyl lithium in diethyl ether at 24°C. was added portionwise over thirty minutes 6.0 g. of 2,7-dimethyl-2,3-dehydro-5-(3-methoxyphenyl)morphanium perchlorate (from Example 3). The reaction mixture was stirred at 24°C. for two hours following complete addition of the salt. The reaction mixture next was diluted by the addition of 30 ml. of saturated aqueous ammonium chloride, and the organic layer then was separated, washed with water and dried. Removal of the solvent by evaporation under reduced pressure afforded 4.79 g. of the product as an oil. The oil was converted to the hydrobromide salt by reaction with hydrogen bromide in diethyl ether. The salt thus formed was recrystallized from 100 ml. of diisopropyl ether and 250 ml. of isopropanol to give 3.18 g. of 2,3,7-trimethyl-5-(3-methoxyphenyl)morphanium bromide. M.P. 191-194°C.

Analysis calc. for $C_{18}H_{28}BrNO$

Theory: C, 61.02; H, 7.97; N, 3.95.

Found: C, 61.01; H, 7.79; N, 4.20.

Example 6

2,3-Dimethyl-5-(3-methoxyphenyl)morphan

Eight grams of 2-methyl-2,3-dehydro-5-(3-methoxyphenyl)morphanium perchlorate were reacted with 75 ml. of 1.6 molar methyl lithium in diethyl ether according to the general procedure set out in Example 5 to give 6.33 g. of 2,3-dimethyl-5-(3-methoxyphenyl)-morphan. B.P. 140-143°C. at 0.1 torr.

The compound thus formed was converted to its hydrobromide salt by reaction with excess hydrogen bromide in diethyl ether. The salt which precipitated was collected and recrystallized twice from 25 ml. of diisopropyl ether and 100 ml. of isopropanol to give 2.79 g. of 2,3-dimethyl-5-(3-methoxyphenyl)morphanium bromide. M.P. 192.5-194°C.

Analysis calc. for $C_{17}H_{24}BrNO$

Theory: C, 60.00; H, 7.77; N, 4.12.

Found: C, 59.87; H, 7.50; N, 4.11.

Example 7

2-Benzyl-5-phenyl-7-ethylmorphan

A solution of 1-benzyl-4-phenyl-1,2,5,6-tetrahydropyridine in tetrahydrofuran containing *n*-butyl lithium is added to a solution of 3-chloro-2-ethylpropene in tetrahydrofuran. The reaction is carried out according to the procedure of Preparation 1 to give 1-benzyl-4-phenyl-4-(2-ethylallyl)-1,4,5,6-tetrahydropyridine. The latter compound is reacted with phosphoric acid and formic acid to give, after treatment with sodium hydroxide, 2-benzyl-3,4-dehydro-5-phenyl-7-ethylmorphan. Catalytic hydrogenation of the latter compound provides 2-benzyl-5-phenyl-7-ethylmorphan.

Example 8

2-Allyl-3-*iso*propyl-5-phenyl-7-ethylmorphan

A solution of 2-benzyl-3,4-dehydro-5-phenyl-7-ethylmorphan in diethyl ether is added to a stirred ethereal solution of *iso*propyl lithium according to

the method of Example 9 to give 2-benzyl-3-isopropyl-5-phenyl-7-ethylmorphan. Catalytic hydrogenation of the latter compound effects debenzylation to provide 3-isopropyl-5-phenyl-7-ethylmorphan. Allyl bromide is reacted with the latter compound to effect N-alkylation to provide 2-allyl-3-isopropyl-5-phenyl-7-ethylmorphan.

## CLAIMS

1. A 2,3-dehydro-5-phenylmorphan compound of the formula

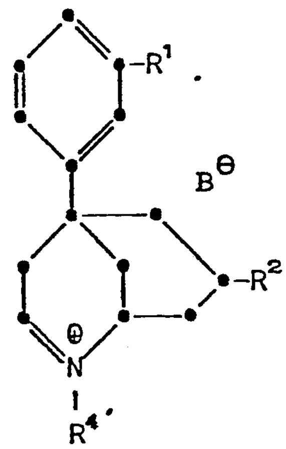

III

wherein

$R^1$ is hydrogen, hydroxy or $C_1$-$C_3$ alkoxy;

$R^2$ is hydrogen, $C_1$-$C_5$ alkyl or $CH_2C_2$-$C_4$ alkenyl;

$R^{4'}$ is hydrogen, $C_1$-$C_{10}$ alkyl, phenyl $C_1$-$C_3$ alkyl, or $CH_2C_3$-$C_6$ cycloalkyl; and

$B^{\ominus}$ is an anion

2. A 3,4-dehydro-5-phenylmorphan compound of the formula

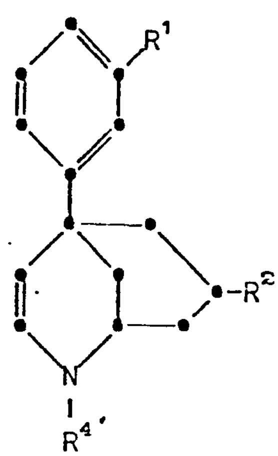

IV

wherein:

$R^1$ is hydrogen, hydroxy or $C_1$-$C_3$ alkoxy;

$R^2$ is hydrogen, $C_1$-$C_5$ alkyl or $CH_2C_2$-$C_4$ alkenyl; and

$R^{4'}$ is hydrogen, $C_1$-$C_{10}$ alkyl, phenyl $C_1$-$C_3$ alkyl or $CH_2C_3$-$C_6$ cycloalkyl.

3. A process for preparing a compound of any one of Claims 1 to 6 characterized by reacting a 1,2,5,6-tetrahydro-4-phenylpyridine compound of the formula

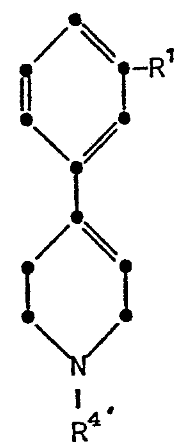

I

with a propenyl alkylating agent of the formula

$XCH_2C-R^2$
$\quad\;\; \| $
$\quad\; CH_2$

wherein:

$R^1$ is hydrogen, hydroxy or $C_1$-$C_3$ alkoxy;

$R^2$ is hydrogen, $C_1$-$C_5$ alkyl or $CH_2C_2$-$C_4$ alkenyl;

$R^{4'}$ is hydrogen, $C_1$-$C_{10}$ alkyl, phenyl $C_1$-$C_3$ alkyl, or $CH_2C_3$-$C_6$ cycloalkyl; and X is a good leaving group in the presence of a strong base to provide a 1,4,5,6-tetrahydro-4-phenyl-4-(2-propenyl)-pyridine compound of the formula

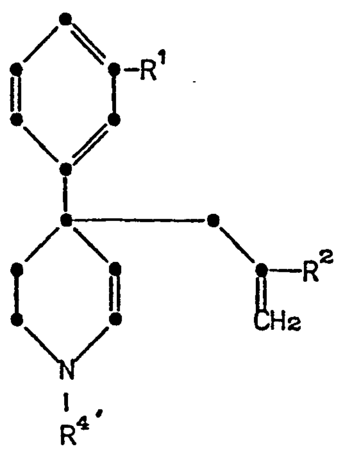

II

wherein:

$R^1$, $R^2$ and $R^{4'}$ are the same as above and reacting said compound with a protonic acid of the formula HB', in which B' is an anion B or B" wherein the product is recovered as the salt in which B' is the anion B or as the free base of a salt in which B is exchanged for the anion B".

European Patent Office

**0059989**

Application number

# EUROPEAN SEARCH REPORT

EP 82 10 2714

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| A | JOURNAL OF MEDICINAL CHEMISTRY, vol. 20, no. 2, February 1977, pages 221-228, Washington, USA M. TAKEDA et al.: "Azabicycloalkanes as analgetics. 3. Structure-acitivity relationships of 1-phenyl-6-azabicyclo(3.2.1)octanes and absolute stereochemistry of (+)-1-(3-Hydroxyphenyl)-6-methyl-6-azabicyclo(3.2.1)octane and its 7-endo-methyl derivative" * Whole document, particularly page 221, right-hand column, paragraph 3 *<br>--- | 1,2 | C 07 D 221/22 //<br>C 07 D 211/70<br>A 61 K 31/435 |
| A | JOURNAL OF MEDICINAL CHEMISTRY, vol. 17, no. 1, January 1974, pages 133-134, Washington, USA H.H. ONG et al.: "Phenylmorphan-agonists-antagonists" * Whole document *<br>----- | 1,2 | TECHNICAL FIELDS SEARCHED (Int. Cl. 3)<br>C 07 D 221/00 |

The present search report has been drawn up for all claims

| Place of search | Date of completion of the search | Examiner |
|---|---|---|
| THE HAGUE | 01-07-1982 | ALLARD M.S. |

CATEGORY OF CITED DOCUMENTS

- X : particularly relevant if taken alone
- Y : particularly relevant if combined with another document of the same category
- A : technological background
- O : non-written disclosure
- P : intermediate document
- T : theory or principle underlying the invention
- E : earlier patent document, but published on, or after the filing date
- D : document cited in the application
- L : document cited for other reasons
- & : member of the same patent family, corresponding document

EPO Form 1503 03.82